# EUROPEAN PATENT APPLICATION

(11) **EP 1 634 497 A1**
(43) Date of publication of application: **15.03.2006**
(21) Application number: 05076436.4
(22) Date of filing: 14.06.2005
(51) Int. Cl.: A01N 1/02, A61F 9/007, B65D 81/22

(54) **Suspension device for donor corneal tissue storage**

(30) Priority: 14.09.2004 EP 04077537
(71) Applicant: Medical Technology Transfer Holding B.V., 3071 MJ Rotterdam (NL)
(72) Inventor: Melles, Gerrit R.J. Med.Tech.Transfer Holding BV, 3071 MJ Rotterdam (NL); Lock, Frank Michael, 3071 MJ Rotterdam (NL)

(57) **Abstract**

This invention relates to a device that is uniquely suited for use in an EyeBank. In more specific embodiments, this invention relates to devices for suspension of a donor cornea or donor corneo-scleral rim in a culture medium. The invention relates to a biocompatible element as a suspension device, so that the device itself does not significantly change or interfere with the culturing process or the clinical transplantation process of the donor cornea.

## Description

### DESCRIPTION OF THE INVENTION

Since the 1970s, several storage methods are available for preservation of donor corneas prior to transplantation. Preservation of donor corneas is performed in EyeBanks, facilities that have the infrastructure and logistics for procurement of the tissue, and subsequent sterile preparation and preservation, most commonly with a culture medium that allows storage of donor corneas up to several weeks using cold storage at 4°C or organ culture at 31°C.

With either storage method, the method to physically position the cornea during preservation differs among Eyebanks. Most often, the cornea is positioned epithelial side down on the bottom of a glass or plastic vial containing medium. A disadvantage of 'free positioning' a donor cornea is that the epithelium may be rubbed off due to the direct contact with the vial or the vial material may form a scaffold for abnormal epithelial outgrowth. As a result, an epithelial defect may be present at the end of the culture period and subsequently in the early postoperative phase.

To overcome this problem, a suture may be used to attach the donor cornea to the lid of the vial, so that the cornea is floating freely in the medium at some distance from the lid, without prlonged and significant contact with the vial itself. As an alternative, various types of plastic holders attached to the lid of the vial or the vial itself are available to position the cornea upon. With all these systems, the handling of the tissue is significantly prolonged, and as a result it may be more difficult to maintain sterility and to avoid tissue damage, and the devices used are relatively expensive.

It is the object of the present invention to describe a device that can be safely used as a suspension device for a donor cornea stored in culture medium, the mechanism by which these devices obtain their effect, and the methods to avoid significant damage to the tissue or interference with the overall function of the tissue and/or medical complications. A first object is a suspension device for keeping the donor cornea afloat in the culture medium. A second object is a suspension device that allows positioning of the donor cornea in a petri dish or the like.

The present invention relates to a device that can quickly and easily be attached to or removed from a donor cornea or most often a corneo-scleral rim (ie. a donor cornea with a rim of sclera still attached). The material and the size and shape of the device are chosen in such a way that the specific gravity of the synthetic material just exceeds the gravity force ie. the weight of the device plus the attached donor tissue. As a result, the suspension device will keep the donor tissue just afloat, while the device as well as the tissue are still immersed in the medium. It is important that the floating capacity of the device is not too strong, because the device will then show a tendency to float, ie. to lay flat on the surface of the medium, thereby distorting the donor tissue or prohibiting complete immersion of the tissue.

The size and thickness of the device is chosen such that it does allow easy tissue handling and positioning of the tissue into a vial or removal of the tissue from a vial. The device can have any desired shape, but preferably the device is 2 to 30 mm in diameter, more preferably 7 to 25 mm in diameter; 0.05 to 5.0 mm in thickness, more preferably 0.1 to 2.0 mm in thickness; and with a specific gravity of <1.0, more preferably between 0.90 to 0.98.

The embodiment of the device includes an attachment means consisting of an opening 1 to 3 mm in diameter bordered by two lips at the outer edge of the device to create a narrow opening, so that the opening serves as a barbed hook. The scleral rim of the donor cornea can be pulled into/over the barbed hook, so that the tissue is 'trapped' and attached to the device. Since only the central corneal tissue is designated for transplantation, ie. the tissue at more than 3 mm from the edge of the corneo-scleral rim, fixating the tissue onto the attachment means will not result in damage to the tissue, since the device is attached to the far periphery of the tissue.

The attachment of the tissue to the device is enhanced by the natural tendency of the tissue to swell. Fixation of the tissue to the device is achieved by the fact that the tissue inside the opening can swell more than at the edge of the device, so that the tissue is trapped inside the opening of the device. However, although the tissue is firmly attached to the device during organ culture, when desired the tissue can be easily removed from the device, for example by the surgeon at the start of the transplantation.

The device is built from an inert material that does not affect the culture medium and that does not induce any significantly inflammatory, hypersensitivity or other type of tissue reaction, if a part of the device contacts or is embedded in the patient tissues during surgery. Suitable materials include but are not limited by foam products and plastics, for example poly-ethylene, polypropylene or a combination thereof. The edge(s) of the device are smoothened to avoid inadvertent damage of the donor tissue or inflammation of the adjacent ocular tissues during surgery. The outer, larger surfaces may have serrations or grooves to allow better grip of instruments holding the device. The device is sterilized with a sterilization method that does not affect the device material(s).

The design of the device may be chosen in such a way that it adds to or alters the floating capacity of the suspension device. For example, the device may contain a space or pocket inside its embodiment, for example it may have a balloon shape. Furthermore, the design and/or the material from which the device is built may contain multiple pockets. The space(s) or pockets within the device may be vacuum or filled with any type of material with a specific gravity lower than 1.0, for example a gas, for example air.

Because the floating capacity (specific gravity) of the suspension device should just exceed the gravity force ie. the weight of the device plus the attached donor tissue, the material from which the device is built, and the dimensions of the device may vary with volume, temperature, atmorpheric pressure, and type and contents of medium used, ie. with the specific gravity of that medium. For example, in a medium with a relatively high specific gravity, the suspension device itself may be smaller, thinner or may have a lower specific gravity, to obtain the same capacity of the suspension device to keep the donor tissue afloat. An organ culture medium as commonly used in EyeBanks consists of Minimal Essential Medium (MEM), Fetal Calf Serum (FCS) and various additives such as antibiotics, buffer, vitamines, dextran, etc. A medium may therefore have any specific gravity, but preferably the specific gravity is between 1.0 and 2.0, and more preferably between 1.0 and 1.2.

Preferred embodiments of the invention are:
1. Under sterile conditions, a corneo-scleral rim, 14-16 mm in diameter is excised from a donor globe. Then, one forceps is used to hold the corneo-scleral rim and another forceps is used to hold the suspension device. The device is than attached to the corneo-scleral rim just adjacent to the forceps holding the rim. The suspension device with the corneo-scleral rim attached can then be positioned in a vial containing culture medium.
2. The corneo-scleral rim is attached to the device as described under 1. At a given point during the organ culture process, under sterile conditions the rim attached to the suspension device is removed from the vial containing culture medium, to allow evaluation of the tissue. The suspension device equiped with stabilization arms may then be used to position the rim in a sterile petri dish. The stabilization arms prevent the device from laying flat onto the bottom of the petri dish and distorting the tissue. As a result, the device does not have to be removed from the tissue but can remain attached to the donor tissue throughout the entire examination.
3. The vial containing the corneo-scleral rim held by the suspension device is then put in a refridgerator or incubator for preservation of the donor tissue at the desired temperature. 3-30 days after culture the vial containing the donor tissue attached to the device may be sent of for transplantation in a recipient patient.
4. At surgery, under sterile conditions the rim attached to the suspension device is removed from the vial containing culture medium, and the suspension device is detached from tissue, to allow further processing of the tissue prior to transplantation into a recipient patient.

For a better understanding of the present invention embodiments of a suspension device, use thereof, methods for attaching donor corneal tissue to the device, and donor corneal tissue preserved therewith, in relation to the accompanying drawings, in which shows:
Figure 1A: The embodiment of the suspension device in frontal view;
Figure 1B: The embodiment of the suspension device with stabilisation arms in frontal view;
Figure 2: In cross section, the suspension device with a donor corneo-scleral rim attached to it;
Figure 3: In cross section, the suspension device with donor tissue attached to it in a vial with preservation medium.
Figure 4: The embodiment of the suspension device containing an air pocket to enable, improve and/or alter the floating capacity of the device.

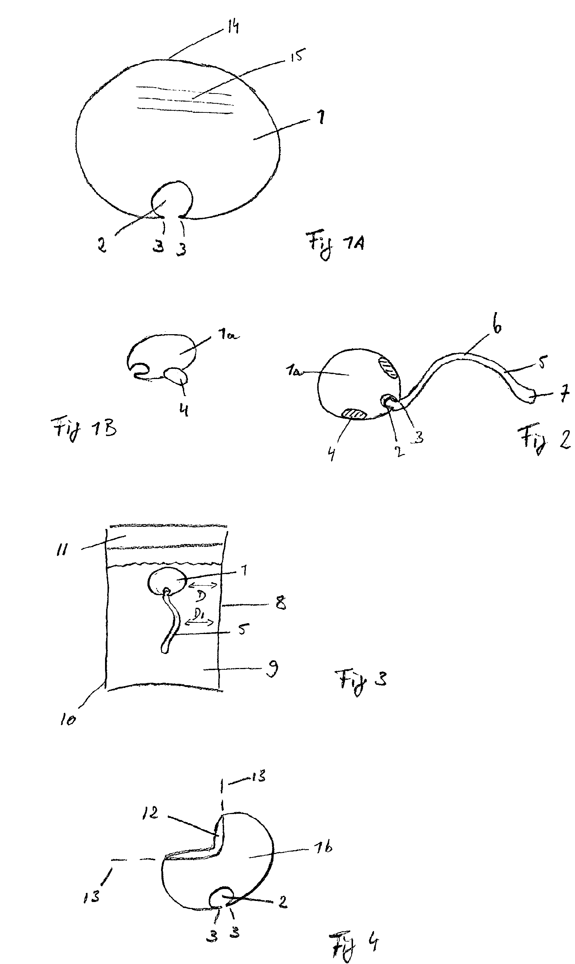

In figure 1A the suspension device is shown, schematically, in frontal view. The device 1 is made of a plastic of foam material or any material with a specific gravity of less than 1.0. The device comprises an attachment means consisting of a hole 2 and two barbed hooks 3 at the peripheral edge of the device by which the scleral tissue can be 'trapped'. The edge 14 is smooth or rounded so that the donor or recipient tissue is not damaged when contacted by the device. To improve the grip of an instrument holding the device, the device may have serrations or grooves 15 on one or more of its larger surfaces.

In figure 1B the suspension device is shown with two stabilisation arms, schematically, in frontal view. The device 1a can have lateral extensions 4 of various shapes and sizes

In figure 2 a corneo-scleral rim 5 consisting of a central cornea 6 and a peripheral scleral rim 7 is attached to the suspension device 1a through the attachment means 2 and 3.

In figure 3 a corneo-scleral rim 5 attached to the suspension device 1 is positioned in a vial 8 containing preservation medium 9. The specific gravity of the suspension device is such that it keeps the donor tissue afloat while most of the device and the tissue are immersed in the medium. The distance between the edge of the suspension device and the vial D is smaller than the distance between the tissue and the vial D1, so that the tissue is not exposed to prolonged contact with the vial.

In figure 4 an embodiment of the suspension device 1b is shown in partial cross section, in which the device 1b contains an air pocket 12 to improve the capacity of the device to float. The dotted lines 13 represent the sectional planes.

## Claims

1. A suspension device, comprising a material with a specific gravity lower than a culture medium used for storage of donor corneas or corneo-scleral rim to keep a donor cornea or a corneo-scleral rims afloat during preservation or prior to transplantation.

2. A suspension device according to claim 1, wherein said material has an attachment means for fixation of tissue to the device.

3. A suspension device according to claim 1 or 2, wherein said material has an attachment means comprising an opening within the device bordered by two lips at the outer edge of the device serving as barbed hooks to trap the tissue in order to fixate the tissue.

4. A suspension device according to claim 1-3, wherein the device is made of a material with a specific gravity of <1.0 and more preferably between 0.90 and 0.98.

5. A suspension device according to any of the previous claims, wherein the size of the device is 2 to 30 mm in diameter and more preferably 7 to 25 mm in diameter; and 0.05 to 5.0 mm in thickness and more preferably 0.1 to 2 mm in thickness.

6. A suspension device according to any of the previous claims, wherein attachment means are provided for permanently or releasably connecting said device to a donor cornea or corneo-scleral rim.

7. A suspension device according to any of the previous claims, wherein the outer edge of the device is substantially rounded.

8. A suspension device according to any of the previous claims, wherein the larger outer surfaces of the device contain serrations or grooves.

9. A suspension device according to any of the previous claims, wherein the device is equiped with two stabilization arms.

10. A suspension device according to any of the previous claims, wherein the device is substantially made of a material or combination of materials such as plastics, foam or any other material with a specific gravity less than 1.0.

11. A suspension device according to any of the previous claims, wherein the device is substantially made of a material or combination of materials such as plastics, foam or any other material with any specific gravity, in which the design of the device is such that it contains one or more vacuum pockets or pocket(s) filled with a substance with a specific gravity of less than 1.0.

12. A suspension device according to any of the previous claims, wherein the device is substantially made of a material or combination of materials such as plastics, foam or any other material that does not interact with or have a hazardous effect on the tissue culture medium, the tissue culturing process or the quality and sterility of the tissue cultured.

13. A suspension device according to any of the previous claims, wherein the material or combination of materials used allows sterilization.

14. A suspension device according to any of the previous claims, enclosed in a closed container like a box or pouch, said suspension device being sterilized or sterilisable within said container.

15. A suspension device according to any of the previous claims, attached to a donor cornea or donor corneo-scleral rim.

16. A suspension device according to any of the previous claims, attached to a donor cornea or donor corneo-scleral rim in a tissue preservation medium.

17. A suspension device according to any of the previous claims, attached to a donor cornea or donor corneo-scleral rim, wherein said preservation medium mainly consists of Minimal Essential Medium (MEM) and Fetal Calf Serum (FCS) for organ culture at 25-40°C and more preferably 29-34°C, or cold storage at 2-8 °C and more preferably 3-5°C.

18. A suspension device according to any of the previous claims, attached to a donor cornea or donor corneo-scleral rim, wherein said preservation medium mainly consists of medium for

19. A suspension device according to any of the previous claims, attached to a donor cornea or donor corneo-scleral rim, wherein said device and donor cornea is positioned in a vial containing preservation medium for organ culture at 25-40°C or cold storage at 2-8°C.

20. Method for attaching a suspension device to a donor cornea or corneo-scleral rim, comprising the steps of:
- providing said device made of a material that does not affect tissue preservation;
- attaching the scleral rim of a donor cornea or donor corneo-scleral rim to the device;
- placement of the device with the attached donor corneal tissue into a tissue preservation medium;
- preservation of donor corneal tissue with the device attached;
- evaluation of donor corneal tissue with the device attached;
- transportation of the donor corneal tissue with the device attached;
- removing the device from the donor corneal tissue before evaluation or at the time of transplantation.
